# EUROPÄISCHE PATENTSCHRIFT

(11) **EP 1 380 311 B1**
(45) Veröffentlichungstag und Bekanntmachung des Hinweises auf die Patenterteilung: **09.03.2005**
(21) Anmeldenummer: 02015377.1
(22) Anmeldetag: 10.07.2002
(51) Int. Cl.: A61L 27/36

(54) **Verfahren zum Herstellen eines Präparats zur Förderung der Regeneration von Knochenstoff**
Multi-step process for making a bone regeneration paste
Procédé de fabrication d'un matériau de régénération pour l'os

(43) Veröffentlichungstag der Anmeldung: 14.01.2004
(73) Patentinhaber: Jakob, Karl, Dipl.-Ing. Dr. h.c., 85598 Baldham (DE); Topor, Boris, Prof. Dr. habil. med., 2045 Chisinau (MD); Topor, Olga, Doz. Ph.D., 2045 Chisinau (MD)
(72) Erfinder: Topor, Boris, Prof. Dr. habil. med., 2045 Chisinau (MD)
(74) Vertreter: Körner, Ekkehard, Dipl.-Ing.

(56) Entgegenhaltungen:
- WO-A-02/45623
- US-A- 4 277 238
- US-B1- 6 311 690

## Beschreibung

Die Erfindung bezieht sich auf das Gebiet der morphoinduktiven Knochentransplantate, die in vielfältigen Bereichen am menschlichen Körper anwendbar sind, insbesondere für plastische Operationen in der Neurochirurgie, in der Otorhinolaryngologie, in der Kiefer- und Gesichtschirurgie, der Traumatologie, Orthopädie und Onkologie.

Knochendefekte am menschlichen Körper sind die Folge von Geburtsfehlern, von Unfällen oder Krankheiten. Solche Defekte können nicht nur aus Fehlstellungen oder Abnutzungen bestehen, sondern auch darin, dass Knochen oder Knochenteile fehlen.

Zur Behebung solcher Defekte gibt es Prothesen oder Ersatzstücke, von denen wohl die Metall- oder Kunststoffplatten für den Ersatz fehlender Teile der Schädeldecke und künstliche Gelenke dem breiten Publikum am meisten bekannt geworden sind. Es ist indessen auch bekannt, dass der Einsatz solcher körperfremden Ersatzstücke keineswegs eine ideale Lösung zur Behebung der entsprechenden Defekte darstellt. Unter medizinischen, insbesondere auch psychologischen Gesichtspunkten wäre einer Reparatur der Defekte durch körpereigene Kräfte, soweit dieses möglich ist, der Vorzug zu geben.

Die Erfindung geht von der Überlegung aus, morphogenetische Proteine, die die Natur für die Regelung des Prozesses der reparativen Osteogenese vorgesehen hat, für die Behandlung der vorgenannten Defekte nutzbar zu maschen.

In diesem Zusammenhang ist aus US-A-4 277 238 ein Verfahren zum Herstellen von artifiziellen Knochen und Zähnen bekannt. Dieses Verfahren sieht folgende Schritte vor. Vernetzungsverarbeitung eines Knochenteils, das von einem Säugetier oder Menschen stammt, mit Formaldehyd, Entsalzung mit Salzsäure, Fettentfemung mit Äthanol und Gefriertrocknung des so bearbeiteten Knochenteils. US-B-6 311 690 beschreibt ein Verfahren zum Herstellen eines bio-kampatiblen Knochenersatzpräparats durch Dispergieren von Knochenwachstum auslösenden Teilen, die aus entrnineralisierter Knochenmatrix oder Gemischen aus entmineralisierter Knochenmatrix und Kollagen bestehen können, in einer wässrigen Lösung eines Vernetzungsmittels, Oberflächenaktivierung oder Teilvernetzung der Teile, Entnehmen der behandelten Teile aus der wässrigen Lösung und Pulverisieren der Teile.

Der Erfindung liegt die Aufgabe zugrunde, ein Verfahren anzugeben, mit dem aus menschlichen Knochen, die einer Knochenbank entnommen sind, ein Präparat hergestellt werden kann, mit dem nach Applikation im menschlichen Körper der induktive Mechanismus der Regeneration und die Differierung der multipatenten Zellen in Knochenzellen in Gang gesetzt werden kann.

Das erfindungsgemäße Verfahren ist im Anspruch 1 beschrieben. Bevorzugte Ausführungsformen davon sind Gegenstand der Unteransprüche. Ein nach der Erfindung hergestelltes Präparat ist Gegenstand des Anspruchs 13.

Das erfindungsgemäße Verfahren wird nachfolgend im Detail erläutert.

Im erfindungsgemäßen Verfahren werden Röhrenknochen verwendet, die einer Knochen bank entnommen werden und von 25- bis 50-jährigen Spendern stammen. In diesem Alter sind die Knochen nämlich besonders reich an morphogenetischen Proteinen. Die Knochen werden mechanisch gereinigt, denn es wird nur das reine Knochenmaterial benötigt. Muskeln, Knochenhaut und Knochenmark müssen entfernt werden.

Um die Knochen besser bearbeiten zu können, zerteilt man sie in kleinere Stücke von etwa 2 bis 4 cm Größe. Dieses hängt von dem Gerät ab, mit dem die Knochenteile später nochmals zerstückelt werden.

Die zerkleinerten Knochenteile werden abgetrocknet, vorzugsweise lediglich mit einem Tuch. Eine vollkommene Durchtrocknung mittels Luft oder Wärmeeinsatz ist nicht erforderlich. Dann werden sie 18 bis 24 Stunden tiefgefroren bei einer Temperatur zwischen -10°C und -40°C, vorzugsweise um etwa -20°C, aufbewahrt, um sie für die nachfolgende Zerteilung in kleine Stücke vorzubereiten und um eine Autolyse der Stoffe zu vermeiden. Sodann werden die Knochenteile in kleine Stücke unterteilt, deren größte Abmessungen 1 mm nicht überschreiten sollten. Bevorzugt sind Abmessungen von 0,5 mm x 0,5mm x 1 mm. Die Größe dieser Knochenstücke ist optimal für das spätere Zusammenwirken des Endprodukts mit dem Blut und anderen Stoffen des behandelten Patienten. Bei der Bearbeitung des Knochenmaterials bei diesem Schritt sollte die Temperatur unter 15°C bleiben, weil die Proteine sonst inaktiviert werden.

Die kleinen Knochenstücke werden dann in einem inerten Behälter mit einer 0,2 bis 0,5%-igen Formaldehydlösung übergossen, wobei das Volumenverhältnis von Formaldehyd zu Knochen zwischen 1 : 5 und 1 : 10 liegen sollte und vorzugsweise bei 1 : 7 liegt. Die verwendete Formaldehydlösung ist vorzugsweise aus einem 35 oder 25%-igen, methanolfreien, extra reinen Formaldehyd gewonnen, das in einer 0,85 bis 0,95%-igen Natriumchloridlösung gelöst worden ist. Diese Behandlung hat den Zweck, die Proteine zu fixieren. Durch die methanolfreie Formaldehydlösung und die Natriumchloridlösung werden nämliche die Proteine fixiert, ohne inaktiviert zu werden.

Die Behandlungsdauer in der Formaldehydlösung beträgt etwa 48 Stunden, wobei die Temperatur zwischen 2°C und 15°C gehalten wird. Dabei ist aber ein Wechsel der Formaldehydlösung nach 1, 2, 4, 6 und 24 Stunden, jeweils gerechnet vom Beginn der Aufbewahrung in der Formaldehydlösung, durchzuführen, weil für die volle Fixierung der Proteine stets frische, freie Formaldehydmoleküle benötigt werden. Zwischen den einzelnen Wechseln der Formaldehydlösung wird der Behälter stündlich für jeweils etwa eine Minute gerüttelt, damit die kleinen Knochenstücke nicht zusammenbacken.

Nach Abschluss dieser Behandlung wird die Formaldehydlösung abgegossen, und es wird eine 1 bis 3%-ige, vorzugsweise 1,5%-ige, chemisch reine Salzsäure eingegossen. Mit dieser werden Phosphor- und Calciumsalze entfernt, die durch ihre Natur die Wirksamkeit der morphogenetischen Proteine blockieren.

Nach einer Einwirkzeit der Salzsäure von 24 +/- 2 Stunden wird die Salzsäure abgegossen und erneut eine 0,2 bis 0,5%-ige, vorzugsweise 0,25%-ige Formaldehydlösung eingegossen, um die Proteine fixiert zu behalten.

Nach einer Einwirkzeit von 24 +/- 2 Stunden wird die Formaldehydlösung abgegossen und erneut eine Salzsäure der vorgenannten Art eingegossen, um den Demineralisierungsprozess fortzusetzen.

Der Wechsel von Formaldehydlösung und Salzsäure wird insgesamt etwa 12 bis 14 mal wiederholt, um immer neue Proteine zu öffnen und zu fixieren. Im Prinzip wird damit ein Demineralisierungsprozess durchgeführt und optimiert. Während dieser gesamten Behandlung wird die Temperatur stets unter 15°C gehalten.

Nach dem letzten Abgießen der Salzsäure wird eine 1%-ige Natruimthiosulfatlösung eingegossen, die man wenigstens 5 min und bis zu 60 min lang einwirken lässt und dann abgießt. Hiermit soll die Wirkung der Salzsäuremoleküle neutralisiert werden.

Nach dem Abgießen der Natriumthiosulfatlösung wird eine 1,5%-ige Ammoniaklösung eingegossen, die man 5 min einwirken lässt und dann abgießt. Hiermit sollen die Moleküle der Formaldehydlösung inaktiviert werden.

Anschließend wird eine 0,01 bis 0,1%-ige Lösung von Chlorhexidin bigluconat eingegossen, die als Grundlage einen 70%-igen Ethylalkohol hat. Diese Lösung lässt man 10 min bis 2 Stunden, vorzugsweise 1 Stunde, einwirken und gießt sie dann ab. Der Zweck dieser Maßnahme ist eine vollständige Sterilisation des Knochenstoffs.

Dann wird ein 70%-iger Ethylalkohol eingegossen, den man nach 4 bis 8 Stunden, vorzugsweise 6 Stunden, und nochmals nach 24 bis 48 Stunden, vorzugsweise 24 Stunden, jeweils vom Beginn des erstmaligen Eingießens, wechselt. Mit dieser Maßnahme werden Fette und fettgebundene Proteine entfernt und die antigene Aktivität des behandelten Produkts reduziert.

Nach 48 Stunden wird der Ethylalkohol abgegossen und an seiner Stelle eine 3,6 bis 3,7%-ige Trisaminlösung (Trisamin = Trioxymethylaminomethan) eingegossen. Diese dient der vollen Detoxikation und der Normalisierung der pH-Werte des Produkts. Die Trisaminlösung wird nach 1 Stunde Einwirkzeit abgegossen, und an ihrer Stelle wird eine 0,9%-ige Natriumchloridlösung eingegossen. Der Grund dafür ist, dass die Proteine in einer Lösung aufbewahrt werden sollen, die neutral für den Körper ist, eine sogenannte isotonische Lösung.

Diese Natriumchloridlösung wird jeweils nach 1, 2, 3, 4, 5, 6 und 24 Stunden, jeweils gerechnet vom erstmaligen Einfüllen, gewechselt und schließlich nach einer weiteren Stunde abgegossen. Sie dient dazu, alle toxischen Produkte vollkommen zu entfernen, die durch die vorangehende Einwirkung der chemischen Stoffe auf das Produkt entstanden sind. Abweichung von den Wechselintervallen der Größenordnung von +/- 10 min sind unschädlich.

Nach dem letzten Abgießen der Natriumchloridlösung lässt man das Produkt abtropfen. Dieses ist nun fertig. Es ist eine Paste, die eine Konstitution wie feuchter Sand hat. Sie ist in gewissem Umfang fließfähig und lässt sich beispielsweise mit geeignet dicken Kanülen spritzen. Diese Paste wird portioniert und verpackt und ist tiefgekühlt aufzubewahren. Ihre höchste Wirksamkeit hat sie in den ersten sechs Monaten nach ihrer Herstellung.

Zur Anwendung wird diese Knochenpaste in die Bruchstelle oder Defektstelle der Knochen durch einen chirurgischen Schnitt oder mit einer Spezialspritze mit dicker Kanüle eingeführt. Der Vorteil der Behandlung mit der in erfindungsgemäßer Weise hergestellten Paste besteht darin, dass in der Matrix des Knochenstoffs alle angeborenen Proteine erhalten sind, nämlich die morphogenetischen, die für den Beginn und die Regelung des Regenerationsprozesses der reparativen Osteogenesis zuständig sind, sowie die nichtmorphogenetischen, die die Knochenregeneration unterstützen. Gleichzeitig werden die antigenen Eigenschaften reduziert, die toxischen Reagenzien neutralisiert und beseitigt.

## Patentansprüche

1. Verfahren zum Herstellen eines Präparats zur Förderung der Regeneration von Knochenstoff, das folgende Merkmale aufweist:
a) Röhrenknochen von 25- bis 50-jährigen Spendern werden von Muskeln, Knochenhaut und Knochenmark befreit, in 2 bis 4 cm große Teile zerteilt und abgetrocknet;
b) die Knochenteile werden auf eine Temperatur von -10°C bis -40°C tiefgekühlt und in diesem Zustand 18 bis 24 Stunden aufbewahrt;
c) die tiefgefrorenen Knochenteile werden in kleine Stücke im Größenbereich (0,1 bis 1) mm x (0,1 bis 1) mm x (0,1 bis 1) mm zerteilt, wobei die Bearbeitungstemperatur unter 15°C gehalten wird;
d) die kleinen Knochenstücke werden in einem inerten Behälter mit einer 0,2 bis 0,5 %-igen Formaldehydlösung mit einem Volumenverhältnis Formaldehydlösung zu Knochen von 1 : (5-10) übergossen, wobei die Formaldehydlösung aus 35 oder 25 %-igem, methanolfreiem, extrareinem Formaldehyd gewonnen ist, das in einer 0,85 bis 0,95 %-igen Natriumchloridlösung gelöst wurde;
e) die Knochenstücke werden in der Formaldehydlösung für 48 Stunden bei einer Temperatur zwischen 2°C und 15°C aufbewahrt, wobei die Formaldehydlösung nach 1, 2, 4, 6 und 24 Stunden, jeweils gerechnet vom Beginn der Aufbewahrung an, gewechselt wird und zwischen den Wechseln der Behälter in Intervallen von jeweils 1 Stunde etwa 1 Minute lang geschüttelt wird;
f) nach Abschluß der Behandlung im Schritt e) wird die Formaldehydlösung abgegossen und eine 1 bis 3 %-ige, chemisch reine Salzsäure eingegossen;
g) nach einer Einwirkzeit der Salzsäure von 24 +/- 2 Stunden wird die Salzsäure abgegossen und erneut eine 0,2 bis 0,5 %-ige Formaldehydlösung eingegossen;
h) nach einer Einwirkzeit der Formaldehydlösung von 24 +/- 2 Stunden wird die Formaldehydlösung abgegossen und wieder eine 1 bis 3 %-ige Salzsäure eingegossen;
i) die Schritte g) und h) werden insgesamt 12 bis 14 mal wiederholt, wobei die Temperatur im Behälter stets unter 15°C gehalten wird;
j) nach dem letzten Abgießen der Salzsäure wird eine 1 %-ige Natriumthiosulfuricumlösung eingegossen, 5 bis 60 Minuten einwirken lassen und dann abgegossen;
k) anschließend wird eine 1,5 %-ige Ammoniakwasserlösung eingegossen, 5 Minuten einwirken lassen und dann abgegossen;
l) sodann wird eine 0,01 bis 0,1 %-ige Lösung von Chlorhexidinbigluconat eingegossen, die als Grundlage einen 70%-igen Ethylalkohol hat, 10 Minuten bis 2 Stunden einwirken lassen und dann abgegossen;
m) dann wird ein 70%-iger Ethylalkohol eingegossen, der nach 4 bis 8 Stunden und nochmals nach 24 bis 48 Stunden, jeweils gerechnet vom Beginn des erstmaligen Eingießens des Ethylalkohols, gewechselt wird;
n) spätestens nach 48 Stunden vom ersten Eingießen wird der Ethylalkohol abgegossen und eine 3,6 bis 3,7%-ige Trisamin (Trioxymethylaminomethan) -Lösung eingegossen;
o) die Trisaminlösung wird nach 1 Stunde Einwirkzeit abgegossen und an ihrer Stelle eine 0,9 %-ige Natriumchloridlösung eingegossen;
p) die Natriumchloridlösung wird nach 1, 2, 3, 4, 5, 6 und 24, jeweils gerechnet vom erstmaligen Einfüllen, gewechselt, wobei Zeitabweichungen bei den ersten 6 Wechseln von jeweils +/- 10 Minuten und beim letzten Wechseln von jeweils +/-1 Stunde zulässig sind;
q) nach einer Einwirkzeit von etwa 1 Stunde nach dem letzten Wechsel der Natriumchloridlösung wird diese abgegossen und das entstandene Produkt wird abtropfen lassen.

2. Verfahren nach Anspruch 1, bei dem die Tiefkühlzeit 24 Stunden beträgt.

3. Verfahren nach Anspruch 1 oder 2, bei dem die Tiefkühltemperatur zwischen -18°C und -20°C liegt.

4. Verfahren nach einem der vorhergehenden Ansprüche, bei dem die Größe der im c) erzeugten Knochenstücke etwa 0,5mm x 0,5mm x 1 mm beträgt.

5. Verfahren nach einem der vorhergehenden Ansprüche, bei dem das Volumenverhältnis von Formaldehyd zu Knochenstücken im Schritt d) etwa 1 : 7 ist.

6. Verfahren nach einem der vorhergehenden Ansprüche, bei dem die Kühltemperatur im Schritt e) zwischen 5°C und 10°C liegt.

7. Verfahren nach einem der vorhergehenden Ansprüche, bei dem die Konzentration der im Schritt g) verwendeten Salzsäure 1,5 % ist.

8. Verfahren nach einem der vorhergehenden Ansprüche, bei dem die im Schritt h) verwendete Formaldehydlösung eine 0,25 °-ige Formaldehydlösung ist.

9. Verfahren nach einem der vorhergehenden Ansprüche, bei dem die Einwirkzeit im Schritt k) etwa 5 Minuten ist.

10. Verfahren nach einem der vorhergehenden Ansprüche, bei dem die Einwirkzeit der Chlorhexidinbigluconatlösung im Schritt im Schritt l) etwa 1 Stunde ist.

11. Verfahren nach einem der vorhergehenden Ansprüche, bei dem die Wechsel des Ethylalkohols im Schritt m) nach 6 und 24 Stunden, jeweils nach dem ersten Einfüllen des Ethylalkohols stattfinden.

12. Verfahren nach einem der vorhergehenden Ansprüche, bei dem die Knochenteile im Schritt a) nur mit einem Tuch abgetrocknet werden.

13. Knochenpaste, die nach dem Verfahren nach einem der vorhergehenden Ansprüche hergestellt ist.

## Claims

1. A method of making a preparation for stimulating the regeneration of osteogenic material, comprising the following features:
a) hollow bones of 25 to 50 year old donors are freed from muscles, periosteum and bone marrow, are divided into fragments of a size of 2 to 4 cm and are dried up;
b) the bone fragments are deep-frozen to a temperature between -10°C to -40°C and are stored in this condition for 18 to 24 hours;
c) the deep-frozen bone fragments are divided into small pieces in the scale of (0.1 to 1) mm x (0.1 to 1) mm x (0.1 to 1) mm, wherein the working temperature is kept below 15°C.
d) the small bone pieces are covered in an inert container by pouring a 0.2 to 0.5% formaldehyde solution with a volume ratio of formaldehyde solution to bone of 1 : (5-10), wherein the formaldehyde solution is obtained from 35 or 25%, methanol-free, extra pure formaldehyde, which was solved in a 0.85 to 0.95% sodium chloride solution;
e) the bone pieces are stored in the formaldehyde solution for 48 hours at a temperature between 2°C and 15°C, wherein the formaldehyde solution is changed after 1, 2, 4, 6 and 24 hours, starting at the beginning of the storage, and the container is shaken for approx. 1 minute in intervals of 1 hours between said changes;
f) after termination of the treatment in step e), the formaldehyde solution is poured off and 1 to 3% chemical pure hydrochloric acid is poured in;
h) after a reaction time of the hydrochloric acid of 24 +/- 2 hours, the hydrochloric acid is poured off and another 0.2 to 0.5% formaldehyde solution is poured in;
h) after a reaction time of the formaldehyde solution of 24 +/- 2 hours, the formaldehyde solution is poured off and another 1 to 3% hydrochloric acid is poured in;
i) steps g) and h) are repeated 12 to 14 times, wherein the temperature in the container is kept constantly below 15°C;
j) after the last pouring off of the hydrochloric acid, a 1% sodium thiosulfuricum solution is poured in, is allowed to react for 5 to 60 minutes to be subsequently poured off;
k) finally, a 1.5% ammonium water solution is poured in, is allowed to react for minutes to be subsequently poured off;
l) then, a 0.01 to 0.1% solution of chlorohexidinebigluconate is poured in, which has a basis of 70% ethyl alcohol, is allowed to react for between 10 minutes and 2 hours to be then poured off;
m) subsequently, 70% ethyl alcohol is poured in, which is changed after 4 to 8 hours and again after 24 to 48 hours, starting with the beginning of the first pouring in of the ethyl alcohol;
n) at the latest 48 hours after the first pouring in, the ethyl alcohol is poured off and 3.6 to 3.7% trisamine (trioxymethylaminomethane) solution is poured in;
o) the trisamine solution is poured off after a reaction time of 1 hour and it is replaced by a 0.9% sodium chloride solution;
p) the sodium chloride solution is changed after 1, 2, 3, 4, 5, 6 and 24 hours after the first filling in, wherein deviations in time in the first 6 changes of +/- 10 minutes and in the last change of +/- 1 hour are allowed;
q) after a reaction time of approx. 1 hour after the last change of the sodium chloride solution, this solution is poured off and the resulting product is let drop.

2. A method as claimed in claim 1, in which the deep-freezing time is 24 hours.

3. A method as claimed in claim 1 or 2, **characterized in that** the deep-freezing temperature is between -18°C and -20°C.

4. A method as claimed in one of the preceding claims, in which the size of the bone pieces formed in step c) is approximately 0.5 mm x 0.5 mm x 1 mm.

5. A method as claimed in one of the preceding claims, in which the volume ratio of formaldehyde to bone pieces in step d) is approximately 1 : 7.

6. A method as claimed in one of the preceding claims, in which the cooling temperature in step e) is between 5°C and 10°C.

7. A method as claimed in one of the preceding claims, in which the concentration of the hydrochloric acid used in step g) is 1.5%.

8. A method as claimed in one of the preceding claims, in which the formaldehyde solution used in step h) is a 25% formaldehyde solution.

9. A method as claimed in one of the preceding claims, in which the reaction time in step k) is approximately 5 minutes.

10. A method as claimed in one of the preceding claims, in which the reaction time of the chlorohexidinebigluconate solution in step l) is approximately 1 hour.

11. A method as claimed in one of the preceding claims, in which the change of the ethyl alcohol in step m) after 6 and 24 hours takes place after the first filling-in of the ethyl alcohol.

12. A method as claimed in one of the preceding claims, in which the bone pieces in step a) are dried by a piece of cloth.

13. An osteogenic paste manufactured according to one of the preceding claims.

## Revendications

1. Procédé de fabrication d'une préparation pour stimuler la régénération de la substance osseuse, présentant les caractéristiques suivantes :
a) des os tubulaires de donneurs âgés de 25 à 50 ans sont débarrassés des muscles, du périoste et de la moelle osseuse, réduits en fragments de 2 à 4 cm et séchés ;
b) les fragments d'os sont congelés à une température de -10°C à -40°C et conservés dans cet état pendant 18 à 24 heures ;
c) les fragments d'os congelés sont réduits en petits morceaux dans le domaine de dimensions de (0,1 à 1) mm × (0,1 à 1) mm × (0,1 à 1) mm, la température de traitement étant maintenue en dessous de 15°C ;
d) les petits morceaux d'os sont recouverts dans un récipient inerte d'une solution de formaldéhyde à 0,2 à 0,5 %, avec un rapport en volume de la solution de formaldéhyde à l'os de 1 : (5-10), la solution de formaldéhyde étant obtenue à partir de formaldéhyde à 35 ou 25 % extra pur exempt de méthanol, qui a été dilué dans une solution de chlorure de sodium à 0,85 à 0,95 % ;
e) les morceaux d'os sont conservés dans la solution de formaldéhyde pendant 48 heures à une température comprise entre 2°C et 15°C, la solution de formaldéhyde étant renouvelée au bout de 1, 2, 4, 6 et 24 heures, calculées à chaque fois à partir du début de la conservation, et le récipient étant agité pendant environ 1 minute entre les renouvellements à des intervalles de 1 heure chacun ;
f) après la fin du traitement de l'étape e), la solution de formaldéhyde est déversée et un acide chlorhydrique chimiquement pur à 1 à 3 % est versé ;
g) après une durée d'action de l'acide chlorhydrique de 24 +/- 2 heures, l'acide chlorhydrique est déversé et une solution de formaldéhyde à 0,2 à 0,5 % est versée à nouveau ;
h) après une durée d'action de la solution de formaldéhyde de 24 +/- 2 heures, la solution de formaldéhyde est déversée et un acide chlorhydrique à 1 à 3 % est versé à nouveau ;
i) les étapes g) et h) sont répétées au total 12 à 14 fois, la température dans le récipient étant maintenue en dessous de 15°C ;
j) après le dernier déversement d'acide chlorhydrique, une solution à 1 % de thiosulfate de sodium est versée, laissée agir pendant 5 à 60 minutes et ensuite égouttée ;
k) ensuite une solution aqueuse à 1,5 % d'ammoniac est versée, laissée agir pendant 5 minutes et égouttée ;
l) ensuite une solution à 0,01 à 0,1 % de bigluconate de chlorohexidine qui a pour base un alcool éthylique à 70 % est versée, laissée agir pendant 10 minutes à 2 heures et ensuite égouttée ;
m) puis est versé un alcool éthylique à 70 % qui est renouvelé au bout de 4 à 8 heures, puis à nouveau au bout de 24 à 48 heures, calculées à chaque fois à partir du début du premier remplissage à l'alcool éthylique ;
n) au plus tard 48 heures après le premier remplissage, l'alcool éthylique est déversé et une solution à 3,6 à 3,7 % de trisamine (trioxyméthylaminométhane) est versée ;
o) la solution de trisamine est déversée au bout de 1 heure et une solution à 0,9 % de chlorure de sodium est versée à sa place ;
p) la solution de chlorure de sodium est renouvelée au bout de 1, 2, 3, 4, 5, 6 et 24 heures, calculées à chaque fois à partir du premier remplissage, des écarts de durée de +/- 10 minutes à chaque fois lors des 6 premiers renouvellements et de +/-1 heure à chaque fois lors du dernier renouvellement étant autorisés ;
q) après une durée d'action d'environ 1 heure après le dernier renouvellement de la solution de chlorure de sodium, celle-ci est déversée et le produit obtenu est égoutté.

2. Procédé selon la revendication 1, dans lequel la durée de congélation est de 24 heures.

3. Procédé selon la revendication 1 ou 2, dans lequel la température de congélation est comprise entre -18°C et -20°C.

4. Procédé selon l'une des revendications précédentes, dans lequel la taille des morceaux d'os produits en c) est d'environ 0,5 mm × 0,5 mm × 1 mm.

5. Procédé selon l'une des revendications précédentes, dans lequel le rapport en volume du formaldéhyde aux morceaux d'os à l'étape d) est d'environ 1 : 7.

6. Procédé selon l'une des revendications précédentes, dans lequel la température de réfrigération de l'étape e) est comprise entre 5°C et 10°C.

7. Procédé selon l'une des revendications précédentes, dans lequel la concentration de l'acide chlorhydrique utilisé à l'étape g) est de 1,5 %.

8. Procédé selon l'une des revendications précédentes, dans lequel la solution de formaldéhyde utilisée à l'étape h) est une solution de formaldéhyde à 0,25 %.

9. Procédé selon l'une des revendications précédentes, dans lequel la durée d'action de l'étape k) est d'environ 5 minutes.

10. Procédé selon l'une des revendications précédentes, dans lequel la durée d'action de la solution de bigluconate de chlorohexidine de l'étape l) est d'environ 1 heure.

11. Procédé selon l'une des revendications précédentes, dans lequel le renouvellement de l'alcool éthylique de l'étape m) s'effectue au bout de 6 et 24 heures, à chaque fois après le premier remplissage à l'alcool éthylique.

12. Procédé selon l'une des revendications précédentes, dans lequel les fragments d'os de l'étape a) sont simplement séchés avec un linge.

13. Pâte d'os qui est fabriquée par le procédé selon l'une des revendications précédentes.
